(19) Europäisches Patentamt
European Patent Office
Office européen des brevets

(11) Publication number : **0 566 237 A2**

(12) **EUROPEAN PATENT APPLICATION**

(21) Application number : **93301730.3**

(51) Int. Cl.$^5$ : **C07K 7/02,** A61K 37/64

(22) Date of filing : **08.03.93**

(30) Priority : **13.03.92 US 852851**

(43) Date of publication of application :
**20.10.93 Bulletin 93/42**

(84) Designated Contracting States :
**AT BE CH DE DK ES FR GB GR IE IT LI LU NL PT SE**

(71) Applicant : **ELI LILLY AND COMPANY
Lilly Corporate Center
Indianapolis Indiana 46285 (US)**

(72) Inventor : **Hui, Kwan Yuk
121 Brunswick Court
Carmel, Indiana 46033 (US)**

(74) Representative : **Tapping, Kenneth George et al
Erl Wood Manor
Windlesham Surrey, GU20 6PH (GB)**

(54) **Retroviral protease inhibitors.**

(57) The present invention provides compounds that inhibit the action of retroviral proteases. These compounds incorporate transition-state residues at the core of low molecular weight peptide analogs and include naphthylalanine residues at both termini that enhance the compounds' inhibitory capacity. Thus, the compounds can be used to inhibit retroviral proteases in a biological solution and prevent the processing of retroviral polyproteins. The compounds can also be used to inhibit the replication and maturation of retroviruses in cell culture or in vivo.

EP 0 566 237 A2

Diseases caused by retroviruses are becoming alarmingly prevalent and have been the recent focus of intensive research efforts. For example, acquired immunodeficiency syndrome (AIDS) has been one of the major health-care concerns of the past several years. The causative agent of the disease is a human retrovirus referred to as Human Immunodeficiency Virus type-1 (HIV-1). HIV-1 uses a reverse transcriptase to synthesize DNA from the viral RNA. The DNA becomes integrated into the host genome and, under the host cell's transcriptional and translational mechanisms, encodes polyprotein precursors known as gag and gag-pol. These proteins are processed to yield the retroviral protease, reverse transcriptase, and endonuclease/integrase. The protease further cleaves the gag protein to yield mature HIV-1 polypeptides known as p6, p7, p17, and p24, which are the building blocks for an intact viral particle.

HIV-1 protease falls within the general class of aspartyl proteases, which demonstrate sequence and structure homology and share a highly conserved catalytic sequence. It has been demonstrated that certain peptide analogs inhibit the proteolytic activity of retroviral proteases where the scissile amide bond has been replaced with nonhydrolyzable transition-state mimetic residues. In turn, these protease inhibitors are useful in treating retroviral infections by interrupting the processing of the viral gene products that function in maturation and replication of the viruses. For review of the rationale for attacking retroviral infections by inhibiting the protease enzyme see Tomaselli, A.G., Howe, W.J., Sawyer, T.K., Wlodawer, A., Heinrikson, R.L., The complexities of AIDS: an assessment of the HIV protease as a therapeutic target, Chimicaoggi, May 1991, pp 6-27; and Debouck, C., and Metcalf, B., Human Immunodeficiency Virus protease: A Target for AIDS Therapy, Drug Dev. Res. 21:1-17 (1990).

The present invention provides a series of compounds which inhibit the action of retroviral proteases and methods of using these compounds to inhibit retroviral proteases in a biological solution. Retroviral proteases function in the maturation and replication of the retrovirus by processing the polyprotein precursors encoded by the viral genome into the necessary mature proteins. Thus, the present invention also provides methods for treating a retroviral infection by administering an antiviral amount of a compound or compounds which are protease inhibitors thereby preventing the maturation and replication of the virus. The present invention further provides pharmaceutical formulations comprising as active ingredient the above mentioned compounds.

In particular, the present invention provides a compound of formula (I):

$$X-NH-\underset{R^1}{\underset{|}{CH}}-\overset{O}{\overset{\|}{C}}-P_2-NH-\underset{R^2}{\underset{|}{CH}}-\overset{OH}{\overset{|}{CH}}-CH_2-\overset{O}{\overset{\|}{C}}-P_2{'}-NH-\underset{R^1}{\underset{|}{CH}}-\overset{O}{\overset{\|}{C}}-Y \qquad (I)$$

wherein:

X is H, ($C_1$-$C_3$ alkyl)-C(O)-, $C_1$-$C_3$ alkyl, or sulfonyl;

Y is OH, $NH_2$, or $C_1$-$C_3$ alkoxy;

$P_2$ and $P_2'$ are independently any naturally or non-naturally occurring amino acids;

each $R^1$ is independently

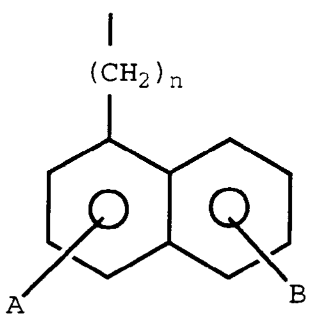

or

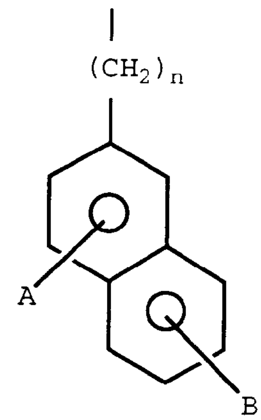

;

A and B are independently H, $C_1$-$C_3$ alkyl, halo, hydroxy, or trifluoromethyl;

n is an integer from 0 to 3; and

$R^2$ is $C_1$-$C_7$ alkyl, ($C_4$-$C_8$ cycloalkyl)-($C_1$-$C_3$ alkyl), phenyl $C_1$-$C_3$ alkyl, or substituted phenyl $C_1$-$C_3$ alkyl;

or a pharmaceutically acceptable salt thereof.

The present invention also provides a method of inhibiting retroviral proteases which comprises adding to a biological solution an inhibitory amount of a compound of formula (I).

The present invention further provides a method of inhibiting viral replication which comprises administering to an animal or cell culture an amount of a compound of formula (I) that inhibits the replication of a retrovirus.

The present invention also provides pharmaceutical formulations comprising as active ingredient a compound of formula (I) together with a pharmaceutical carrier.

For purposes of the present invention, as disclosed and claimed herein, the following terms and abbreviations are as defined below.

Terms and Abbreviations:

Aba - an aminobutyric acid residue
AHPPA - a 4-amino-3-hydroxy-5-phenylpentanoic acid residue
Boc - tert-butyloxycarbonyl
FITC - fluorescein isothiocyanate
Fmoc - 9-fluorenylmethoxycarbonyl
NA - naphthylalanine
2NA - beta-naphthylalanine
Nle - a norleucine residue
Nva - a norvaline residue
Sta - statine, a 4-amino-3-hydroxy-6-methylheptanoic acid residue

Naturally occurring amino acids are indicated by their standard three letter codes.

The compounds of the current invention have the ability to inhibit the proteolytic activity of the aspartyl proteases, including retroviral proteases such as HIV protease, and are, therefore, potential therapeutic agents for fighting retroviral infections.

Retroviral proteases generally display a symmetrical structure about the enzyme's catalytic site. HIV-1 protease, for example, exists as a homodimer displaying a $C_2$-symmetrical structure. The enzyme's catalytic site comprises hydrophobic pockets at subsites of the enzyme designated $S_3$ and $S_3$'. The compounds of the current invention incorporate transition-state residues at the core of short peptide analogs and include naphthylalanine residues at both the $P_3$ and $P_3$' positions of the analogs to enhance the hydrophobic interaction of the inhibitors with the enzyme and increase their inhibitory capacity. The result is a series of compounds that are potent inhibitors of the HIV aspartyl protease and, therefore, are potential therapeutic agents for fighting HIV infection.

A study of the relationship between potential inhibitors' structures and their inhibitory capacity proved the presence of a naphthyl ring structure at both the $P_3$ and $P_3$' positions contributes significantly to the ability of low molecular weight peptides to inhibit retroviral proteases. Alpha and beta forms of the naphthyl ring are equally preferred. A single methylene group is most preferred as the connector of the naphthyl rings to the backbone of compounds of formula (I), that is, when n is 1.

Amino acid residues in the formula (I) compounds are referred to by their locus in relation to the transition-state residue which itself is said to be at the $P_1P_1$' position, $P_1$ referring to the amino portion of the transition-state residue and $P_1$' referring to its carboxyl portion. The residue immediately connected to the amino end of the $P_1P_1$' transition-state residue is in the $P_2$ position while the residue immediately connected to the carboxyl end of the residue is in the $P_2$' position. The residue appearing at the amino terminus of the $P_2$ residue is the $P_3$ residue and the residue at the carboxy terminus of the $P_2$' residue is the $P_3$' residue.

With regard to the range of structures that are possible at the $P_1P_1$' position, phenylmethyl and isobutyl sidechains at $R^2$ of formula (I) are preferred. The residues that appear at the $P_2$ and $P_2$' positions are not critical to the activity of the formula (I) compounds and may be selected from any of the naturally or non-naturally occurring amino acids.

Formula (I) compounds that have an acetyl protecting group at X are preferred, as are compounds having an amide protecting group at Y.

The term "alkyl" refers to saturated straight or branched-chain aliphatic hydrocarbon radicals such as methyl, ethyl, propyl, butyl, pentyl, hexyl, heptyl and isomeric forms of these radicals.

When $R^2$ is substituted phenylalkyl, appropriate substituents on the phenyl include $C_1$-$C_3$ alkyl, $C_1$-$C_3$ alkoxy, halo, hydroxyl, trifluoromethyl, and the like. The term "$C_1$-$C_3$ alkoxy" refers to methoxy, ethoxy, and propoxy.

The compounds of this invention form pharmaceutically acceptable acid and base addition salts with a wide variety of organic and inorganic acids and bases. Such salts are also part of this invention. Typical inorganic acids used to form such salts include hydrochloric, hydrobromic, hydroiodic, nitric, sulfuric, phosphoric, hypophosphoric and the like. Organic acids commonly used to form such salts include glycollic, maleic, hydroxymaleic, fumaric, malic, tartaric, citric, salicylic, o-acetoxybenzoic, nicotinic, isonicotinic, methanesulfonic, ethanesulfonic, 2-hydroxyethanesulfonic, p-toluenesulfonic, benzenesulfonic, naphthalene-2-sulfonic, acetic, succinic, cinnamic, benzoic, ascorbic, mandelic, trifluoroacetic, hippuric, oxalic, p-bromophenylsulfonic, carbonic and the like.

Bases commonly used for formation of salts include ammonium hydroxide and alkali and alkaline earth metal hydroxides, carbonates and bicarbonates, as well as aliphatic and aromatic amines, aliphatic diamines and hydroxy alkylamines. Bases especially useful in the preparation of addition salts include ammonium hydroxide, potassium carbonate, sodium bicarbonate, calcium hydroxide, methylamine, diethylamine, ethylene diamine, cyclohexylamine and ethanolamine. The pharmaceutically acceptable salts generally have enhanced solubility characteristics compared to the compound from which they are derived, and thus are often more amenable to formulation as liquids or emulsions.

The formula (I) compounds are useful antiviral agents. For example, in Example 2, typical formula (I) compounds were tested for the ability to inhibit the hydrolysis of a synthetic substrate by recombinant HIV-1 protease. The protease was expressed in E. coli and purified as reported in Margolin, N., Heath, W., Osborne, E., Lai, M., and Vlahos, C., Substitutions at the P2' site of gag p17-p24 affect cleavage efficiency by HIV-1 protease, Biochem. Biophys. Res. Comm. 167:554-560 (1990) and Lai, M.-H. T., Dee, A.G., Zervos, P.H., Heath, W.F., and Scheetz, M.E., Biosynthesis and processing of the gag and pol polyproteins of human immunodeficiency virus type 1 in Escherichia coli, in Retroviral proteases, Maturation and Morphogenesis, pp. 63-71, Pearl, L.H., ed., Stockton, N.Y. (1990). Other methods of acquiring HIV-1 protease in quantities sufficient for testing hydrolytic activity are described in the art.

The assay used to measure the inhibitory activity of the formula (I) compounds uses the concept of particle concentration fluorescence, that is, the binding of a fluorescent moiety to a solid particle followed by concentration and isolation of the particles from the reaction solution and quantitation of the signal using epifluorescence. Specifically, the peptide gly-ser-gln-asn-tyr-pro-ile-val-gly-lys (SEQ ID NO:1), which contains a sequence homologous to the cleavage site segment between p17 and p24 of the HIV-1 gag protein, was synthesized by solid-phase peptide synthesis techniques using standard protocols. Biotin was coupled to the amino terminal glycine, and fluorescein isothiocyanate was then reacted with the carboxy terminus of the biotin-peptide. The protease was preincubated with serial concentrations of peptide inhibitor followed by incubation with the synthetic substrate. The reaction mixture was then mixed with an excess of avidin-coated beads. As a result, the biotinylated peptide became immobilized onto the bead, which was then washed and collected by filtration on a 96-well Pandex Fluoricon Assay Plate (Idexx, Westbrook, ME).

When the synthetic substrate is cleaved by the protease the biotinylated amino terminal fragment is retained by the membrane while the FITC-containing carboxy terminal fragment flows through the membrane. However, in the presence of an HIV-1 protease inhibitor, substrate cleavage is reduced and the resulting intact substrate molecules, including the fluorescent marker, bind to the avidin-coated beads and are retained by the membrane. Thus, an increase of fluorescence, measured in this instance by a Pandex Model 784 Screen Machine (Idexx, Westbrook, ME) and compared to the incubation mixture without inhibitor, indicates an inhibitory effect.

The compounds of formula (I) were found to be effective retroviral protease inhibitors. Thus, the present invention also provides a method of inhibiting retroviral proteases thereby preventing the maturation of the gag precursor polypeptide which may be present in a wide variety of biological solutions. For example, an antiviral amount of any of the compounds of formula (I) can be added to a sample of human blood, serum, or plasma to inhibit any retroviral protease that may be present in the sample. The gag polypeptide could then be more readily removed as a contaminant from the biological solution.

In addition to the HIV-1 protease inhibitory activity, the compounds of formula (I) are also useful for inhibiting the growth of viruses in cell culture and in vivo. For example, several of the compounds were tested for antiviral effect against HIV-1 and were found to significantly increase the survival rates of cells infected with the virus. The results of these tests of antiviral potency are shown in Example 3, Table 2.

Other typical retroviruses that cause infections which can be prevented or treated by the methods of the present invention are: Murine leukemia Virus (MLV), the C-type retrovirus that causes lymphosarcoma in Northern Pike, the C-type retrovirus that infects mink, the caprine lentivirus that infects sheep, Equine Infectious Anemia Virus (EIAV), the C-type retrovirus that infects pigs, Avian leukosis Sarcoma Virus (ALSV), Feline leukemia Virus (FeLV), Feline Aids Virus, Bovine leukemia Virus (BLV), Simian leukemia Virus (SLV), Simian Immunodeficiency Virus (SIV), Human T-cell leukemia Virus type-I (HTLV-I), Human T-cell leukemia Virus type-II (HTLV-II), and Human Immunodeficiency virus type-2 (HIV-2). Those skilled in the art will note that the methods of the present invention would be useful in treating both HIV-1 and HIV-2 infections, as the amino

acid sequence of the HIV-2 protease is 73% homologous to the protease of HIV-1.

Skilled artisans will recognize that the antiviral activity displayed in cell culture by the compounds used in the present method is predictive of the utility of the compounds for use in vivo.

When used in the methods or compositions of this invention, the formula (I) compounds can be administered parenterally (intravenously, intramuscularly or subcutaneously), by inhalation, by the intranasal route, or by other known methods to a mammal suffering from a retroviral infection or susceptible thereto. For example, for parenteral administration, as by the intraperitoneal route, the compound may be dissolved or suspended in water containing 2% of a surface active agent, particularly an emulfor (a polyhydroxylated fatty acid). For intravenous administration, the compound can be dissolved in one of the commonly used intravenous fluids such as physiological saline, Ringer's solution, or 5% dextrose solution and the like. For intramuscular preparations a sterile formulation containing a suitable salt form of the compound (for example, the hydrochloride salt or sodium salt) can be prepared with a suitable vehicle. Examples of such sterile formulations are a suitable salt form-either dissolved in a pharmaceutical diluent (for example, Water-for-Injection, physiological saline, 5% glucose) or suspended in an aqueous base or a pharmaceutically acceptable oil base (for example, an ester of a long chain fatty acid such as ethyl oleate).

To practice the antiviral method of this invention, all that is required is that an antiviral amount of a formula (I) compound be added to the tissue culture to be protected, or be administered to an animal suffering from, or susceptible to, a viral infection. The compounds will ideally be formulated with pharmaceutically acceptable diluents for convenient administration, for example, parenterally, and can be employed for prophylactic as well as therapeutic treatment. The formulations will normally contain from about 1 to about 95% by weight of active antiviral agent.

For severe viral infections, the antiviral compounds will be formulated for intravenous or intramuscular administration. Such formulations will contain from about 1 to about 50% active agent. The compounds will be dissolved in common diluents such as isotonic saline or dextrose solutions for intravenous infusion and can be dissolved in polyhydric aliphatic alcohols such as propylene glycol or polyethylene glycol for easy intravenous or intramuscular injection.

The antiviral agents described above are active over a wide range of dose levels. While the particular dose to be administered will be determined by the precise viral infection to be treated or guarded against and its severity, the route of administration, and related circumstances that will be determined by attending medical practitioners, the normal dose will range from about 0.1 to about 100 mg/kg.

In a preferred method of treatment, the compounds are administered to mammals susceptible to infection with retrovirus including horses, mice, pigs, sheep, and humans. Among humans, the compounds are administered prophylactically, particularly to persons who are at risk of a retroviral infection.

The retroviral protease inhibitors used in this invention can be prepared by any of a variety of recognized peptide synthesis techniques including classical (solution) methods, solid-phase methods, and semisynthetic methods. In the solid-phase technique, an amino acid which will ultimately correspond to the C-terminal amino acid residue of the resulting peptide chain is anchored to an insoluble support resin by means of a covalent linkage formed between the carboxyl group of the C-terminal residue and a specific functional group present on the resin matrix as a site of attachment. The peptide chain is then formed, beginning at the resin-coupled C-terminal amino acid, by sequentially coupling an amino acid to the free N-terminus of the growing peptide chain until the desired amino acid sequence is obtained. Alternatively, small peptide fragments can be prepared and introduced into the peptide chain in the desired order. The peptide chain remains attached to the resin throughout the synthesis but may be cleaved from the resin once the peptide chain is assembled.

The amino acids are coupled using techniques well known in the art for the formation of peptide bonds. One method involves converting the amino acid to a derivative that will render the carboxyl group more susceptible to reaction with the free N-terminal amino group of the growing peptide chain. For example, the amino acid can be converted to a mixed anhydride by reaction of a protected amino acid with ethyl chloroformate, phenyl chloroformate, sec-butyl chloroformate, or isobutyl chloroformate. Alternatively, the amino acid can be converted to an active ester such as a pentafluorophenyl ester, a 2,4,5-trichlorophenyl ester, a p-nitrophenyl ester, an N-tert-butyloxycarbonyl ester, an N-hydroxysuccinimide ester, or an ester formed from 1-hydroxybenzotriazole.

Another coupling method involves use of a suitable coupling agent, such as N,N'-dicyclohexylcarbodiimide (DCC) or N,N'-diisopropylcarbodiimide (DIC). Other appropriate coupling agents will be apparent to those skilled in the art. See Schroder and Lubke, The Peptides, Academic Press, 1965, Chapter III and U.S. Patent 4,259,234.

It should be recognized that the alpha-amino group of each amino acid employed in the peptide synthesis must be protected during the coupling reaction to prevent side reactions involving the reactive alpha-amino function It should also be recognized that certain amino acids contain reactive side-chain functional groups

(e.g. sulfhydryl, epsilon-amino, beta- and gamma-carboxyl, imidazole, guanidino, and hydroxyl) and that such functional groups must also be protected both during the initial and subsequent coupling steps. Suitable protecting groups are known in the art. See, for example, Protective Groups in Organic Chemistry, M. McOmie, ed., Plenum Press, N.Y., 1973 and U.S. Patent 4,617,149.

In selecting a particular protecting group, certain conditions must be observed. An alpha-amino protecting group must render the alpha-amino function inert under the conditions employed in the coupling reaction, it must be readily removable after the coupling reaction under conditions that will not remove side chain protecting groups and which will not alter the structure of the peptide fragment, and the alpha-amino protecting group must eliminate the possibility of racemization upon activation immediately prior to coupling. A side chain protecting group must render the side chain functional group inert under the conditions employed in the coupling reaction, it must be stable under the conditions employed in removing the alpha-amino protecting group, and it must be readily removable, if necessary, upon completion of the peptide under conditions that will not alter the structure of the peptide chain.

It will be apparent to those skilled in the art that the protecting groups known to be useful for peptide synthesis will vary in reactivity to the agents employed for their removal. For example, certain protecting groups, such as triphenylmethyl and 2-(p-biphenylyl)isopropyloxycarbonyl are very labile and can be cleaved under mild conditions. Other protecting groups, such as t-butyloxycarbonyl, t-amyloxycarbonyl, adamantyloxycarbonyl, and p-methoxybenzyloxycarbonyl, are less labile and require moderately strong acids, such as trifluoroacetic, hydrochloric, or boron trifluoride in acetic acid, for their removal. Still other protecting groups, such as benzyloxycarbonyl, halobenzyloxycarbonyl, p-nitrobenzyloxycarbonyl, cycloalkyloxycarbonyl, and isopropyloxycarbonyl are even less labile and require stronger acids, such as hydrogen fluoride (HF), hydrogen bromide, or boron trifluoroacetate in trifluoroacetic acid, for their removal. 9-Fluoroenylmethyloxycarbonyl protecting groups may also be used and are readily removed by piperidine under conditions which leave other protecting groups intact.

Upon completion of the coupling steps, the protected peptide may be cleaved from the resin support and all protecting groups may be removed. The cleavage reaction and removal of the protecting groups may be accomplished simultaneously or stepwise. When the resin support is a chloromethylated polystyrene resin, the bond anchoring the peptide to the resin is an ester linkage formed between the free carboxyl group of the C-terminal moiety and one of the many chloromethyl groups present on the resin matrix. It will be recognized that the anchoring bond can be cleaved by reagents which are known to be capable of breaking an ester linkage and of penetrating the resin matrix. One especially convenient method is by treatment with liquid anhydrous HF. This reagent not only will cleave the peptide from the resin but may also remove all protecting groups. Hence, use of this reagent will directly afford the fully deprotected peptide. When it is desired to cleave the peptide without removing protecting groups, the protected peptide-resin can undergo methanolysis to give the protected peptide in which the C-terminal carboxyl group is methylated. The methyl ester can then be hydrolyzed under mild, alkaline conditions to give the free C-terminal carboxyl. The protecting groups on the peptide chain then can be removed by treatment with a strong acid, such as liquid HF. A particularly useful technique for the creation of peptides like those of this invention is described in Hui, K.Y. et al., 1988, J. Med. Chem. 31, 1679-1686.

Another method for cleaving the protected peptide from the resin is by ammonolysis or by treatment with hydrazine. It will also be recognized that the protecting group present on the N-terminal alpha-amino group may be removed preferentially either before or simultaneously with the cleavage of the protected peptide from the resin support.

Many naturally occurring and non-naturally occurring amino acids are commercially available, in both the protected and unprotected form, from a wide variety of sources. Other amino acids which might not be available from commercial sources may be synthesized. All of the reagents and materials necessary for peptide synthesis are available from Advanced ChemTech (PO Box 1403, Louisville, KY) or Applied Biosystems (850 Lincoln Center Dr., Foster City, CA) unless specified otherwise.

The techniques and sources set forth in the Examples are provided as a means of illustrating the invention and are not to be construed as a limitation thereon.

Preparation 1

Synthesis of N-(tert-Butyloxycarbonyl)-3-(1'-naphthyl)-2(S)-amino-propionic acid (Boc-naphthylalanine):

3-(1'-Naphthyl)alanine (2.3 mmol; 0.5g) was dissolved in a mixture consisting of 4 ml dioxane, 4 ml water, and 1.28 ml of triethylamine (TEA) (9.22 mmol). Ditert-butyl-dicarbonate (3.46 mmol; 0.76 g) was then added. After stirring for 10 hours, the mixture was evaporated to dryness in vacuo. The residue was redissolved in

20 ml of water and extracted three times with 10 ml of hexane. The aqueous layer was acidified with saturated $KHSO_4$ when precipitation occurred. This suspension was extracted with ethyl acetate. The organic layer was isolated, dried with $MgSO_4$, filtered, and then concentrated in vacuo. The product was precipitated from petroleum ether. Final yield = 0.66g (90% of theoretical).

Example 1

Synthesis of Peptides

In general, the retrovirus protease inhibitory peptides of the present invention were synthesized using the classic Merrifield method of stepwise solid phase peptide synthesis. Detailed explanations of the various reactions (such as preparation of Boc Amino Acids, chromatography, ninhydrin tests, etc.) can be found in Stewart and Young, 1984, Solid Phase Peptide Synthesis Second Edition (Pierce Chemical Company). Synthesis of each of the peptides was started with the coupling of the first protected-amino acid to the p-methylbenzhydrylamine resin (United States Biochemical, Cleveland, Ohio). The addition of each Boc-amino acid was accomplished by adding 2.5 equivalents of the Boc-amino acid to the resin, except for the addition of Boc-statine or Boc-AHPPA (Advanced ChemTech, Louisville, Kentucky), when only 1.25 equivalents were used. Boc-statine, Boc-AHPPA or their variants can be produced in substantial accordance with the teaching of Hui et al. 1987, J. Med. Chem. 30:1287-1295 and Boger et al., 1985, J. Med. Chem. 28:1779. The stepwise synthesis of acetyl-β-naphthylalanine-valine-statine-leucine-β-naphthylalanine-amide (Ac-2NA-val-sta-leu-2NA-$NH_2$) was performed as follows:

A. Preparation of Resin and Incorporation of Boc-Amino Acids

Before adding the Boc-amino acid, 0.45 g of p-methylbenzhydrylamine HCl resin (containing 0.33 mmol of the free amino group) was neutralized three times with 10 ml (each wash) 5% N,N-diisopropylethylamine (DIEA, from Aldrich, Milwaukee, Wisconsin) in dichloromethane (DCM) followed by three washes with 10 ml (each wash) DCM. In general, DCM was used as a solvent for the Boc-amino acids and coupling agents. Therefore, 5 ml of DCM containing 2.5 equivalents of Boc-β-naphthylalanine (Boc-2NA) (Peninsula Laboratories, Belmont, CA) was added to the washed resin. N,N'dicyclo-hexylcarbodiimide (DCC) (from Fluka, New York, New York) was used as a condensing agent, therefore, 1 ml of DCM containing 2.5 equivalents of DCC was added to the coupling reaction. This mixture was stirred and allowed to react at room temperature for one to three hours with occasional stirring. A small aliquot was removed, washed with DCM, and tested for the completeness of coupling of the Boc-amino acid to the resin. One drop of a 70% phenol solution (in EtOH) was added to the peptide/resin in a test tube. One drop of a pyridine solution (containing 0.0002 M potassium cyanide) was added followed by one drop of a ninhydrin solution (0.28 M in EtOH). The test mixture was heated to 110°C for two minutes. A blue or amber color indicates the presence of free amino groups while a light yellow color indicates the absence of free amino groups.

Alternatively, the ninhydrin test can be performed upon this small aliquot as follows. The aliquot is washed twice with a solution of 5% TEA in DCM, then three times with DCM, followed by drying in vacuo. Two to five mgs are weighed out and placed into a test tube. 0.1 ml KCN (treated with Amberlite MB-3 (Sigma, St. Louis, MO) to remove ammonia), 0.04 ml ninhydrin and 0.05 ml phenol (also treated with MB-3) are added to the dried resin. This solution is heated to 100°C for 10 minutes, then chilled in cold water. One ml of 60% ethanol is added, then the solution is mixed and filtered through a glass wool plug. The tube is rinsed twice with 0.2 ml of tetraethylammonium chloride (0.5 M) in DCM, then the combined filtrates and washes are brought up to 2.0 ml with 60% ethanol. The absorbance is read at 570 nm against a reagent blank. A light color indicates a high percentage of coupling. The ninhydrin test is adapted from Steward and Young, supra, and Kaiser et al., 1970, Anal. Biochem., 34:595.

B. Removal of the Boc protecting group

Following the coupling reaction, the resin was washed three times with 10 ml (each wash) DCM. Next, 15 ml of 50% trifluoroacetic acid (TFA) in DCM were added and the mixture was stirred for one minute. The supernatant was removed from the resin and another 15 ml of 50% TFA in DCM were added. Stirring continued for 30 minutes. The supernatant was removed and the resin was again washed three times with 10 ml (each wash) DCM. A small aliquot of the resin was removed and the ninhydrin test was performed to monitor the reaction.

C. Neutralization

Before the next Boc-amino acid was coupled to the peptide/resin, the peptide resin was neutralized by stirring in 15 ml of a DIEA solution (5% DIEA in DCM) for one minute. The supernatant was removed and another 15 ml of the DIEA solution was added and the resin was stirred for five minutes. This supernatant was removed, the peptide resin was washed six times with DCM, and the next Boc-amino acid solution was added to the resin.

Steps A, B, and C were sequentially repeated using the appropriate Boc-protected amino acids (commercially available from Peninsula Laboratories, San Carlos, California) to produce a completed peptide/resin having the sequence Boc-β-naphthylalanine-valine-statine-leucine-β-naphthylalanine-resin.

The only change in the procedure occurred during the Boc-statine coupling. When adding Boc-statine, 1.25 equivalents of the amino acid and 1.25 equivalents of the condensing reagent DCC were used. The coupling reaction for Boc-statine was allowed to run for 3 or more hours. Alternatively, larger excesses of Boc-statine or longer coupling times can be used.

D. Acetylation

To the neutralized β-naphthylalanine-valine-statine-leucine-β-naphthylalanine-resin was added sufficient acetic anhydride (for example, 5 ml) to cover the peptide-resin, followed by adding 1 ml of 5% DIEA in DCM. The mixture was stirred and the reaction was allowed to run for 15 min. After that, the resin was washed three times with DCM and subjected to the ninhydrin test as usual. A light yellow color resulted from the ninhydrin test show the amino terminus of the peptide was protected by an acetyl group.

E. Cleavage of Peptide from Resin

The peptide acetyl-β-naphthylalanine-valine-statine-leucine-β-naphthylalanine-amide (Ac-2NA-val-sta-leu-2NA-$NH_2$) was released from the resin by stirring for 45 minutes at 0°C in a solution of 10 parts (v/v) HF (Mateson, Secaucus, New Jersey) containing 10% anisole to 1 part resin. After removal of most of the HF by vacuum the mixture was washed with anhydrous ether. The free peptide was extracted from the residue with glacial acetic acid, 50% acetic acid, and then water. The extracts were pooled and lyophilized. The peptide was purified by reverse-phase high performance liquid chromatography.

The peptide gave the following amino acid composition upon hydrolysis: val, 1.0(1); leu, 1.1(1); Sta, present; 2NA, 1.8(2); which corresponds to the proper ratios for Ac-2NA-val-sta-leu-2NA-$NH_2$. The peptide was also analyzed by fast atom bombardment mass spectrometry which result confirmed the molecular weight (822) of the peptide.

Example 2

The peptide gly-ser-gln-asn-tyr-pro-ile-val-gly-lys (SEQ ID NO:1) was synthesized on an Advanced ChemTech Model 200 Peptide Synthesizer using standard protocols.

Following addition of the amino terminal glycine, the Boc group was removed with trifluoroacetic acid, the resin neutralized with diisopropylethylamine and then biotin was exhaustively coupled to the glycine using dicyclohexylcarbodiimide and 1-hydroxybenzotriazole. The biotin-peptide was cleaved from the resin with 9:1 HF/m-cresol for one hour at 0° C and the residue extracted first with diethyl ether followed by 50% aqueous acetic acid. Following lyophilization, the crude peptide was purified on a 2.2 x 25 cm octadecyl-silica reverse phase column using a linear gradient of 7.5 to 25% $CH_3CN$ in 0.1% $CF_3COOH$. Column fractions containing the desired material were pooled, frozen and lyophilized. Final yield = 1.21 g (62% of theoretical). Amino acid and fast atom bombardment mass spectrometry analyses were consistent with the expected structure

1.21 g of the biotinylated peptide (0.94 mmoles) was dissolved in 100 ml of 0.1M sodium borate, pH.5 with stirring. Then, 3 g of fluorescein isothiocyanate (7.7 mmoles) was dissolved in 15 ml of dimethyl sulfoxide and added to the peptide solution in ten equal portions over the course of two hours. The reaction was allowed to proceed for an additional hour after the last addition of reagent and the solution was then acidified to pH 3 with 5N HCl. The precipitate which formed was removed by centrifugation. The pH of the supernatant was raised to 7.8 with the addition of 5N NaOH and the resulting solution filtered through a 0.22 micron filter and loaded onto a 2.2 x 25 cm. column of octadecyl-silica resin which had been equilibrated with 0.1M ammonium acetate, pH 7.5. The peptide was then eluted from the column using a linear gradient of 5 to 25% $CH_3CN$ in 0.1M ammonium acetate, pH 7.5. The appropriate column fractions were pooled, frozen and lyophilized. Final yield = 190 mg (12% of theoretical). Amino acid and fast atom bombardment mass spectrometry analyses were

consistent with the expected structure.

20 µL of a 27 U/ml of HIV-1 protease (1 U enzyme = amount of protein required to hydrolyze 1 µmol of H-ser-gln-asn-tyr-pro-ile-val-OH per minute at 37° C) in MES-BSA buffer (0.05M MES, pH 5.5, 0.02M NaCl, 0.002M EDTA, 0.001M dithiothreitol, 2.0 mg/ml bovine serum albumin) was added to each well of a 96-well polystyrene microtiter plate. Six µl of potential inhibitory compound in dimethyl sulfoxide was added and the mixture incubated for one hour at 22° C. Then, 20 µl of a 1.50 µg/ml solution of Biotin-gly-ser-gln-asn-tyr-pro-ile-val-gly-lys(FITC)-OH in MES-BSA was added to each well and the plate was incubated for eight hours at 22° C. The reaction was diluted by the addition of 154 µL of MES-BSA and 25 µL of the diluted solution was added to each well of a 96-well Pandex Fluoricon Assay Plate. Immediately prior to the addition of the reaction solutions to the Pandex plate, 25 µL of a 0.1% solution of Fluoricon Avidin Assay Particles in TBSA (0.02M Tris, pH 7.5, 0.15M NaCl, 1.0 mg/ml bovine serum albumin) had been added to each of the wells. The contents of the wells were mixed and the plate loaded into the Pandex Model 784 Screen Machine. Unbound fluorescence was removed by filtration and subsequent washing with TBSA. Sample detection was performed by excitation at 485 nm and reading the resulting epifluorescence at 535 nm.

The results are shown in Table 1. $IC_{50}$ values, reported in nanomolar units as an average of at least three experiments, reflect the concentration of peptide inhibitor that gives a fluorescence intensity halfway between a substrate blank (reaction mixture containing no protease) and an enzyme control (protease plus substrate but no inhibitor).

Table 1

| | Compound | $IC_{50}$ (nM) |
|---|---|---|
| P1 | Ac-NA-val- sta -leu-NA-$NH_2$ | 4.5 |
| P2 | Ac-NA-val- sta -gly-NA-$NH_2$ | 6.1 |
| P3 | Ac-NA-val- sta -ala-NA-$NH_2$ | 3.5 |
| P4 | Ac-NA-val- sta -val-NA-$NH_2$ | 3.5 |
| P5 | Ac-NA-val- sta -ile-NA-$NH_2$ | 4.5 |
| P6 | Ac-NA-val- sta -nva-NA-$NH_2$ | 2.3 |
| P7 | Ac-NA-nva- sta -nva-NA-$NH_2$ | 4.8 |
| P8 | Ac-NA-nva-AHPPA-nva-NA-$NH_2$ | 6.3 |
| P9 | Ac-NA-val- sta -nle-NA-$NH_2$ | 5.9 |
| P10 | Ac-NA-val- sta -aba-NA-$NH_2$ | 4.3 |
| P11 | NA-val- sta -nva-NA-$NH_2$ | 2.7 |
| P12 | NA-val- sta -nva-NA-OH | 1.8 |
| P13 | Ac-NA-val- sta -nva-NA-OH | 1.7 |
| P14 | NA-val-AHPPA-leu-NA-$NH_2$ | 2.4 |
| P15 | Ac-2NA-val- sta -leu-2NA-$NH_2$ | 7.3 |

The peptides synthesized were analyzed for amino acid composition upon hydrolysis and for molecular weight by fast atom bombardment mass spectrometry. The results are shown in Table 2.

Table 2

| Peptide | Residue | Amino Acid Analysis Theo. | Actual | Molecular Weight Theo. | MH+ |
|---|---|---|---|---|---|
| P1 | VAL | 1.00 | 1.02 | 823 | 824 |
| | LEU | 1.00 | 1.01 | | |
| | NA | 2.00 | 1.95 | | |
| | STA | 1.00 | PRES | | |
| P2 | VAL | 1.00 | 1.11 | 767 | 768 |
| | GLY | 1.00 | 0.90 | | |
| | NA | 2.00 | 1.99 | | |
| | STA | 1.00 | PRES | | |
| P3 | VAL | 1.00 | 1.04 | 780 | 781 |
| | ALA | 1.00 | 1.00 | | |
| | NA | 2.00 | 1.96 | | |
| | STA | 1.00 | PRES | | |
| P4 | VAL | 2.00 | 2.03 | 808 | 809 |
| | NA | 2.00 | 1.97 | | |
| | STA | 1.00 | PRES | | |
| P5 | VAL | 1.00 | 1.08 | 822 | 823 |
| | ILE | 1.00 | 1.08 | | |
| | NA | 2.00 | 1.85 | | |

| | | | | | |
|---|---|---|---|---|---|
| | STA | 1.00 | PRES | | |
| P6 | VAL | 1.00 | 1.01 | 808 | 809 |
| | NVA | 1.00 | 1.16 | | |
| | NA | 2.00 | 1.82 | | |
| | STA | 1.00 | PRES | | |
| P7 | NA | 2.00 | 2.14 | 808 | 809 |
| | NVA | 2.00 | 1.85 | | |
| | AHPPA | 1.00 | PRES | | |
| P8 | NA | 2.00 | 2.08 | 842 | 843 |
| | NVA | 2.00 | 1.92 | | |
| | STA | 1.00 | PRES | | |
| P9 | VAL | 1.00 | 1.00 | 822 | 823 |
| | NA | 2.00 | 1.97 | | |
| | NLE | 1.00 | 1.03 | | |
| | STA | 1.00 | PRES | | |
| P10 | VAL | 1.00 | 1.01 | 794 | 795 |
| | ABA | 1.00 | 0.98 | | |
| | NA | 2.00 | 2.01 | | |
| | STA | 1.00 | PRES | | |
| P11 | VAL | 1.00 | 0.98 | 766 | 767 |
| | NA | 2.00 | 1.95 | | |
| | NVA | 1.00 | 1.07 | | |
| | STA | 1.00 | PRES | | |

| | | | | | |
|---|---|---|---|---|---|
| P12 | VAL | 1.00 | 1.06 | 767 | 768 |
| | NA | 2.00 | 1.61 | | |
| | NVA | 1.00 | 1.32 | | |
| | STA | 1.00 | PRES | | |
| P13 | VAL | 1.00 | 0.79 | 809 | 810 |
| | NA | 2.00 | 1.86 | | |
| | NVA | 1.00 | 1.35 | | |
| | STA | 1.00 | PRES | | |
| P14 | VAL | 1.00 | 0.88 | 814 | 815 |
| | NA | 2.00 | 1.93 | | |
| | LEU | 1.00 | 1.12 | | |
| | AHPPA | 1.00 | PRES | | |
| P15 | VAL | 1.00 | 1.01 | 822 | 823 |
| | LEU | 1.00 | 1.14 | | |
| | NA | 2.00 | 1.84 | | |
| | STA | 1.00 | PRES | | |

PRES - present

Example 3

The antiviral assay, using human T lymphoblastoid CEM and MT2 cells, was based on a measurement of cell viability. Cells were infected with HIV-1 by incubation with the cell-free culture medium from a stock virus-producer culture for approximately four hours and subsequently separated from unabsorbed virus by centrifugation at 600 x g for five minutes. The cells were incubated at 37°C in a humidified atmosphere of 5% $CO_2$ in air. Uninfected control cells were similarly treated. The HIV-infected and the control cells were both suspended in fresh RPMI 1640 medium containing antibiotics and supplemented with 10% fetal bovine serum at a density of 1 x $10^5$ cells/ml. Aliquots (0.1 ml) were plated in the appropriate wells of a 96-well culture plate with infected cells being plated in triplicate virus control wells and in triplicate wells for each tested drug concentration. Uninfected control cells were plated in triplicate cell control wells (no virus and no drug) and in duplicate wells for each tested concentration of drug for determination of cytotoxicity. Drug, dissolved in DMSO, was diluted in complete RPMI 1640 medium such that the highest drug concentration used (100 µg/ml) resulted in a nontoxic DMSO concentration (0.25%) upon addition of 0.1-ml aliquots to the appropriate wells.

After incubation at 37°C for 5-7 days, cell viability was measured by addition of 20 µl of a 450 µg/ml stock solution of a tetrazolium salt, MTT (3-[4,5-dimethylthiazol-2-yl]-2,5-diphenyltetrazolium bromide), to each well. After incubation for approximately four hours, 50 µl of 20% aqueous SDS solution was added to each well and incubation was continued for 16-18 hours. Optical densities were recorded at 570 nm using a $V_{max}$ plate reader (Molecular Devices, Inc., Palo Alto, California).

The optical density value is a function of the amount of colored formazan product produced by the metabolism of MTT by viable cells. Data analysis included appropriate corrections for optical densities caused by drug and/or medium alone. The $ID_{50}$ was calculated by a regression analysis program for semilog curve fitting, and is that micromolar concentration of drug which inhibits the HIV-induced lytic cytopathic effect by 50%. The $TD_{50}$ values were calculated in a similar manner and is that micromolar concentration of drug which causes a 50% reduction of viability of uninfected cells maintained under identical culture condition as the infected cells.

The therapeutic index (TI) equals the $TD_{50}$ value divided by the $ID_{50}$ value for a given compound. These results are reported in Table 3.

Table 3

| | CEM | | | MT2 | | |
|---|---|---|---|---|---|---|
| | $ID_{50}$ | $TD_{50}$ | TI | $ID_{50}$ | $TD_{50}$ | TI |
| P1 | Tox | >130 | >111 | 0.36 | >121 | >336 |
| P2 | 1.17 | >130 | >111 | 6.49 | >130 | >20 |
| P3 | Tox | -- | -- | 4.59 | >127 | >28 |
| P4 | Tox | -- | -- | 6.2 | >123 | >20 |
| P5 | Tox | -- | -- | 2.3 | >121 | >53 |
| P6 | Tox | -- | -- | 2.06 | >121 | >59 |
| P7 | 0.39 | 6.16 | 15.6 | 0.65 | 8.62 | 13.2 |
| P8 | 0.41 | 11.8 | 28.6 | 0.53 | 63.5 | 120 |
| P10 | <0.4 | >131 | >328 | 23.6 | >131 | >5.6 |
| P11 | 2.48 | >13 | >5.2 | 4.4 | >13 | >3.0 |
| P13 | 14.8 | >124 | >8.4 | >12 | -- | -- |
| P14 | 0.64 | 27 | 42.2 | 1.7 | >41.7 | >25 |
| P15 | <0.39 | >122 | >313 | 1.72 | >66 | >38 |

```
SEQUENCE LISTING

(1) GENERAL INFORMATION:

     (i) APPLICANT:
          (A) NAME: ELI LILLY & CO
          (B) STREET: LILLY CORPORATE CENTER
          (C) CITY: INDIANAPOLIS
          (D) STATE: IN
          (E) COUNTRY: USA
          (F) ZIP: 46285

    (ii) TITLE OF INVENTION: RETROVIRAL PROTEASE INHIBITORS

   (iii) NUMBER OF SEQUENCES: 1

    (iv) COMPUTER READABLE FORM:
          (A) MEDIUM TYPE: Floppy disk
          (B) COMPUTER: Macintosh
          (C) OPERATING SYSTEM: Macintosh 7.0
          (D) SOFTWARE: Microsoft Word

    (vi) CURRENT APPLICATION DATA:
          (A) APPLICATION NUMBER:

(2) INFORMATION FOR SEQ ID NO:1:

     (i) SEQUENCE CHARACTERISTICS:
          (A) LENGTH:10 amino acids
          (B) TYPE:amino acid
          (D) TOPOLOGY:linear

    (xi) SEQUENCE DESCRIPTION: SEQ ID NO:1:

Gly Ser Gln Asn Tyr Pro Ile Val Gly Lys
 1               5                   10
```

## Claims

**1.** A compound of formula (I):

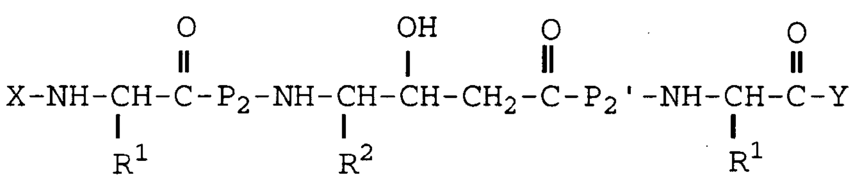

(I)

wherein:
X is H, ($C_1$-$C_3$ alkyl)-C(O)-, $C_1$-$C_3$ alkyl, or sulfonyl;
Y is OH, $NH_2$, or $C_1$-$C_3$ alkoxy;
$P_2$ and $P_2$' are independently any naturally or non-naturally occurring amino acids;
each $R^1$ is independently

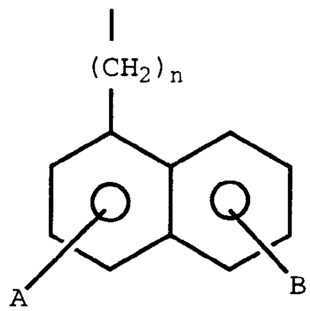

or

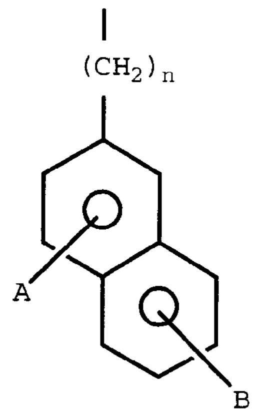

;

A and B are independently H, $C_1$-$C_3$ alkyl, halo, hydroxy, or trifluoromethyl;
n is an integer from 0 to 3; and
$R^2$ is $C_1$-$C_7$ alkyl, ($C_4$-$C_8$ cycloalkyl)-($C_1$-$C_3$ alkyl), phenyl $C_1$-$C_3$ alkyl, or substituted phenyl $C_1$-$C_3$ alkyl;
or a pharmaceutically acceptable salt thereof.

**2.** A compound of claim 1 wherein $R^1$ is

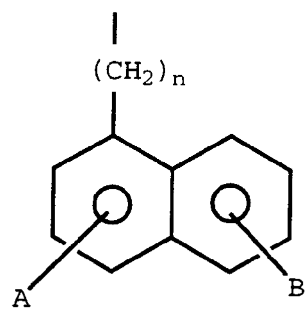

.

**3.** A compound of claim 1 wherein $R^1$ is

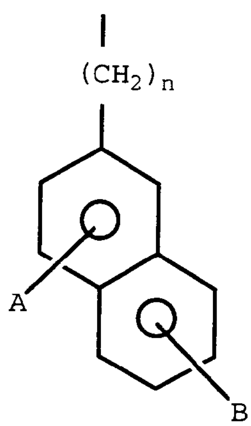

.

**4.** A compound of claim 2 wherein X is acetyl, Y is $NH_2$, and $R^2$ is isobutyl.

**5.** A method of inhibiting retroviral proteases which comprises adding to a biological solution an inhibitory amount of a compound of formula (I) as claimed in any one of claims 1 to 4.

**6.** A method of inhibiting viral replication which comprises administering to an animal or cell culture an amount of a compound of the formula (I) as claimed in any one of claims 1 to 4.

7. A pharmaceutical formulation comprising as active ingredient a compound of formula (I) as claimed in any one of claims 1 to 4.